# EUROPEAN PATENT SPECIFICATION

(11) **EP 1 845 983 B1**
(45) Date of publication and mention of the grant of the patent: **07.04.2010**
(21) Application number: 05820879.4
(22) Date of filing: 24.10.2005
(51) Int. Cl.: A61K 31/445, A61K 31/4535, A61K 9/00

(54) **OPHTHALMIC COMPOSITIONS AND METHODS OF USING THE SAME**
OPHTHALMISCHE ZUSAMMENSETZUNGEN UND VERFAHREN ZU DEREN VERWENDUNG
COMPOSITIONS OPHTALMIQUES ET METHODES D'UTILISATION DE CES COMPOSITIONS

(30) Priority: 25.10.2004 US 972571; 29.10.2004 US 623758 P
(43) Date of publication of application: 24.10.2007
(73) Proprietor: BAUSCH & LOMB INCORPORATED, Rochester, New York 14604 (US)
(72) Inventor: GREEN, Kenneth, E., Duluth, GA 30097 (US); MINNO, George, E., Suwanee, GA 30024 (US); CABALLA, Susan, Sugar Hill, GA 30518 (US)
(74) Representative: Maiwald, Walter
(86) International application number: PCT/US2005/038186
(87) International publication number: WO 2006/047418

(56) References cited:
- WO-A-01/07049
- WO-A-01/47521
- WO-A-99/51230
- US-B2- 6 395 756
- DATABASE WPI Week 198803 Derwent Publications Ltd., London, GB; AN 1988-016591 XP002466600 & JP 62 277323 A (SANKYO CO LTD) 2 December 1987 (1987-12-02)

## Description

### FIELD

The invention generally relates to ophthalmic compositions containing ketotifen and/or a ketotifen salt and methods of using the same.

### BACKGROUND

Various ophthalmic compositions are known for treating allergic conjunctivitis. For example, U.S. Patent No. 6,274,626 is directed towards compositions comprising the antihistamine pheniramine in combination with povidone for preventing and treating ophthalmic allergic responses. Solutions according to U.S. Patent No. 6,274,626 may contain buffers, various surfactants, stabilizers, isotonic agents and the like which aid in making ophthalmic compositions more comfortable to the user. The aqueous solutions of U.S. Patent No. 6,274,626 are typically adjusted with tonicity agents to approximate the osmotic pressure of normal lachrymal fluids, which is stated to be equivalent to a 0.9% solution of sodium chloride or a 2.5% solution of glycerol. An osmolality of about 225 to 400 mOsm/kg is preferred for the solutions, and is more preferably 280 to 320 mOsm/kg. U.S. Patent No. 6,274,626 also states that excess salt or other tonicity agent may result in the formation of a hypertonic solution that will cause stinging and eye irritation.

Ophthalmic compositions for treating allergic conjunctivitis that contain ketotifen are also known. For example, U.S. Patent Nos. 6,774,137 and 6,777,429 relate to an ophthalmic composition comprising ketotifen as a pharmaceutically active agent, comprising a ketotifen salt in a concentration of 0.01 to 0.04%, a non-ionic tonicity agent in an amount such that the total tonicity of the composition has an osmolarity in the range of 210 to 290 milliosmoles, optionally a preservative, an acid or base for bringing the pH to weak acidity, and water. The patents disclose that the ophthalmic composition can be used for the treatment and the temporary prevention of itching of the eye due to allergic conjunctivitis. The patents also disclose that glycerol is the preferred non-ionic tonicity agent and that if glycerol is used, the concentration is preferably in the range of 1.5 to 2.5%.

One commercially available product for temporary prevention of itching of the eye due to allergic conjunctivitis, Zaditor^{™} ketotifen fumarate ophthalmic solution, is a sterile ophthalmic solution containing 0.0345% ketotifen fumarate (equivalent to 0.025% ketotifen), 0.01% benzalkonium chloride, glycerol, sodium hydroxide/hydrochloric acid (to adjust pH), and purified water. The product has a pH of 4.4 to 5.8 and an osmolality of 210-300 mOsm/kg.

### SUMMARY

In one aspect, an ophthalmic composition is provided that consists essentially of (a) ketotifen or a ketotifen salt in a concentration of from 0.01% to 0.05%; (b) a non-ionic tonicity agent in a concentration such that the composition has an osmolality of from 400 to 875 milliosmoles/Kg; and (c) water, and optionally includes an anti-redness agent.

In another aspect, an ophthalmic composition is provided that consists essentially of (a) ketotifen or a ketotifen salt in a concentration of from 0.01% to 0.05%; (b) glycerol in a concentration of from 3.5% to 7%; and (c) water, and optionally includes and anti-redness agent.

In yet another aspect, an ophthalmic composition is provided that consists essentially of (a) ketotifen or a ketotifen salt in a concentration of from 0.01% to 0.05%; (b) glycerol in a concentration of greater than 3.5% such that the composition has an osmolality of from 400 to 875 milliosmoles/Kg; and (c) water, and optionally includes an anti-redness agent.

In another aspect, an ophthalmic composition is provided that comprises (a) ketotifen or a ketotifen salt in a concentration of from 0.01% to 0.05%; (b) a non-ionic tonicity agent in a concentration such that the composition has an osmolality of from 400 to 875 milliosmoles/Kg; and (c) water.

In a further aspect, an ophthalmic composition is provided that comprises (a) ketotifen or a ketotifen salt in a concentration of from 0.01% to 0.05%; (b) glycerol in a concentration of from 3.5% to 7%; and (c) water.

In yet a further aspect, an ophthalmic composition is provided that comprises (a) ketotifen or a ketotifen salt in a concentration of from 0.01% to 0.05%; (b) glycerol in a concentration of greater than 3.5% such that the composition has an osmolality of from 400 to 875 milliosmoles/Kg; and (c) water.

In another aspect, an ophthalmic composition for treating allergic conjunctivitis is provided comprising administering to a subject suffering from or susceptible to allergic conjunctivitis an effective amount of an ophthalmic composition comprising (a) ketotifen or a ketotifen salt in a concentration of from 0.01% to 0.05%; (b) a non-ionic tonicity agent in a concentration such that the composition has an osmolality of from 400 to 875 milliosmoles/Kg; and (c) water.

In yet another aspect, an ophthalmic composition for treating allergic conjunctivitis is provided comprising administering to a subject suffering from or susceptible to allergic conjunctivitis an effective amount of an ophthalmic composition comprising (a) ketotifen or a ketotifen salt in a concentration of from 0.01% to 0.05%; (b) glycerol in a concentration of from 3.5% to 7%; and (c) water.

In a further aspect, an ophthalmic composition for treating allergic conjunctivitis is provided comprising administering to a subject suffering from or susceptible to allergic conjunctivitis an effective amount of an ophthalmic composition comprising (a) ketotifen or a ketotifen salt in a concentration of from 0.01% to 0.05%; (b) glycerol in a concentration of greater than 3.5% such that the composition has an osmolality of from 400 to 875 milliosmoles/Kg; and (c) water.

In another aspect, an ophthalmic composition for treating dry eye disease is provided comprising administering to a human subject suffering from dry eye disease an effective amount of an ophthalmic composition comprising (a) ketotifen or a ketotifen salt in a concentration of from 0.01% to 0.05%; (b) a non-ionic tonicity agent in a concentration such that the composition has an osmolality of from 400 to 875 milliosmoles/Kg; and (c) water.

In yet another aspect, an ophthalmic composition for treating dry eye disease is provided comprising administering to a human subject suffering from dry eye disease an effective amount of an ophthalmic composition comprising (a) ketotifen or a ketotifen salt in a concentration of from 0.01% to 0.05%; (b) glycerol in a concentration of from 3.5% to 7%; and (c) water.

In a further aspect, an ophthalmic composition for treating dry eye disease is provided comprising administering to a human subject suffering from dry eye disease an effective amount of an ophthalmic composition comprising (a) ketotifen or a ketotifen salt in a concentration of from 0.01% to 0.05%; (b) glycerol in a concentration of greater than 3.5% such that the composition has an osmolality of from 400 to 875 milliosmoles/Kg; and (c) water.

### DETAILED DESCRIPTION

The present invention relates to ophthalmic compositions containing ketotifen and/or a ketotifen salt as well as methods of using the same.

Conventional aqueous ophthalmic solutions are typically adjusted with tonicity agents to approximate the osmotic pressure of normal lachrymal fluids, which, as stated in U.S. Patent No. 6,274,626, is equivalent to a 2.5% solution of glycerol. Excess tonicity agent is typically thought, as also stated in U.S. Patent No. 6,274,626, to result in the formation of a hypertonic solution that will cause stinging and eye irritation. Surprisingly, it has been discovered that increasing the osmolality of and/or increasing the concentration of glycerol in ophthalmic compositions containing ketotifen (or a salt thereof) results in greater comfort, a cooling sensation, and/or less stinging, burning, or irritation due to the ophthalmic composition.

The ophthalmic compositions of the present invention comprise ketotifen or a ketotifen salt, a non-ionic tonicity agent, and water. In some embodiments, the ophthalmic compositions consist essentially of ketotifen or a ketotifen salt, a non-ionic tonicity agent, and water. The compositions may include a preservative, may include an acid or base to adjust the pH of the composition, may include a buffer to achieve (and maintain) the desired pH of the compositions, and may also include an anti-redness agent to relieve redness in the eye.

The ketotifen or ketotifen salt is present in the composition in a concentration of 0.01% to 0.05%, preferably 0.01% to 0.04%, more preferably 0.02% to 0.03% (as used herein, "concentration" of a component of an ophthalmic composition means -concentration based on mass of the component per total volume of the composition (i.e., g/mL), and is typically expressed as a percentage). Any ophthalmically acceptable ketotifen salt may be used, although ketotifen fumarate is preferred. Ketotifen fumarate is represented by the following formula: 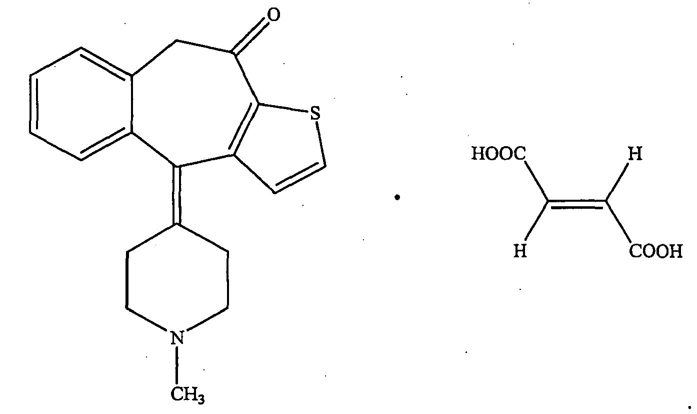 In some embodiments, the ketotifen or ketotifen salt is provided in a concentration such that the concentration of ketotifen base in the composition is 0.02% to 0.03%, preferably 0.0225% to 0.0275%, more preferably 0.025%. Concentrations of ketotifen salts yielding such concentrations of ketotifen base may be readily calculated; for example, using ketotifen fumarate in a concentration of 0.0345% in the composition provides a concentration of ketotifen base in the composition of 0.025%.

The non-ionic tonicity agent is preferably glycerol, although other non-ionic tonicity agents may be used such as, for example, urea, sorbitol, mannitol, propylene glycol, and dextrose. In some embodiments, the non-ionic tonicity agent is provided in a concentration such that the composition has an osmolality from 400 to 875 milliosmoles/kilogram (mOsm/Kg), preferably from 425 to 825 mOsm/Kg, more preferably from 425 to 775 mOsm/Kg, more preferably from 550 to 750 mOsm/Kg, even more preferably from 600 to 725 mOsm/Kg, and yet even more preferably from 650 to 700 mOsm/Kg. In other embodiments, glycerol is used as the non-ionic tonicity agent in a concentration of from 3.5% to 7%, preferably from 4.5% to 7%, more preferably from 5 % to 7%, even more preferably from 5.5.% to 6.5%, and yet even more preferably from 5.75% to 6.25%. In further embodiments, glycerol is used as the non-ionic tonicity agent in a concentration of greater than 3.5%, preferably greater than 4.5%, more preferably greater than 5%, and even more preferably greater than 5.5%. In yet other embodiments, glycerol is used as the non-ionic tonicity agent in a concentration of greater than 3.5%, preferably greater than 4.5%, more preferably greater than 5.5%, even more preferably from 5% to 7%, and yet even more preferably from 5.5% to 6.5%, such that the composition has an osmolality from 400 to 875 mOsm/Kg, preferably from 425 to 825 mOsm/Kg, more preferably from 425 to 775 mOsm/Kg, more preferably from 550 to 750 mOsm/Kg, even more preferably from 600 to 725 mOsm/Kg, and yet even more preferably from 650 to 700 mOsm/Kg.

As stated above, the compositions may include a preservative. A preservative is preferred when the composition is packaged for multidose units, but may be absent from the composition if desired (e.g., in single dose units of the composition). Any preservative may be used with the compositions, but benzalkonium chloride is preferred. Other preservatives that may be used include Polyquad preservative (Alcon); perborate (e.g., sodium perborate from Ciba); Purite preservative (stabilized chlorine dioxide) (Allergan); other quaternary ammonium compounds such as benzoxonium chloride; alkyl-mercury salts of thiosalicylic acid such as, for example, thiomersal, phenylmercuric nitrate, phenylmercuric acetate, and phenylmercuric borate; parabens such as, for example, methylparaben or propylparaben; alcohols such as, for example, chlorobutanol, benzyl alcohol, and phenyl ethanol; guanidine derivatives such as, for example, chlorhexidine or polyhexamethylene biguanide; and the like. When a preservative is used in the composition, the preservative is typically provided in a concentration of 0.005% to 0.02%, preferably 0.01%, although other concentrations may be used.

The ophthalmic compositions typically have a pH from 4 to 6, preferably from 4.4. to 5.8, although the compositions may also have a pH outside of these ranges. A buffer (e.g., buffers including citrates, phosphates, borates, bicarbonates, sodium salts, potassium salts, etc.; or a buffer with intrinsic antimicrobial properties such as a sodium borate/boric acid buffer) may be used to achieve (and maintain) the desired pH of the compositions, and/or an acid or base may be added to adjust the pH of the compositions to the desired level. Typically, only small amounts of an acid or base will be needed to adjust the pH of the composition. The preferred acid and base for adjusting the pH are hydrochloric acid and sodium hydroxide. When the ophthalmic compositions include both ketotifen or ketotifen salt and an anti-redness agent (e.g., naphazoline), it is preferred that the compositions include a buffer.

The ophthalmic compositions may also include an anti-redness agent to relieve redness in the eye. The preferred anti-redness agent is naphazoline or an ophthalmically acceptable salt thereof such as, for example, naphazoline hydrochloride. Other-anti-redness agents that may be used include, but are not limited to, tetrahydrozoline, ephedrine, phenylephrine, other vasoconstrictors, combinations thereof, as well as ophthalmically acceptable salts thereof (e.g., tetrahydrozoline hydrochloride).

In some embodiments, the compositions are free or substantially free of stabilizers such as ethylene diamine tetraacetic acid (EDTA) and salts thereof, Dequest, and Desferal (e.g., as used in compositions described in U.S. Patent Nos. 6,776,982 and 6,468,548); polymers comprising chitosan (e.g., as used in compositions described in U.S. Patent Application No. 2003/0031718); linear polysaccharide compounds such as hyaluronic acid compounds (e.g., as used in compositions described in International Publication No. WO 02/100437); biocompatible polymers/thickeners such as polyoxyethylene-polyoxypropylene copolymers and acrylic acid homo- and co-polymers (e.g., as used in compositions described in International Publication No. WO 02/100436); antioxidants; and/or active agents other than ketotifen. Preferably, the compositions consisting essentially of ketotifen or a ketotifen salt, a non-ionic tonicity agent, and water are free or substantially free of these components.

As stated above, in some embodiments, the ophthalmic composition comprises ketotifen or a ketotifen salt, a non-ionic tonicity agent, and water, and optionally includes a preservative, an anti-redness agent, and/or an acid or base to adjust the pH of the composition. In other embodiments, the ophthalmic composition consists essentially of ketotifen or a ketotifen salt, a non-ionic tonicity agent, and water, and optionally includes a preservative, an anti-redness agent, and/or an acid or base to adjust the pH of the composition. In further embodiments, the composition consists of ketotifen or a ketotifen salt, a non-ionic tonicity agent, and water, and optionally includes a preservative, an anti-redness agent, and/or an acid or base to adjust the pH of the composition. In yet other embodiments, the composition consists of ketotifen or a ketotifen salt, a non-ionic tonicity agent, and water. In yet further embodiments, the composition consists of ketotifen or a ketotifen salt, a non-ionic tonicity agent, an anti-redness agent, and water.

In one particularly preferred embodiment, the ophthalmic composition consists essentially of ketotifen fumarate in a concentration of 0.0345%, glycerol in a concentration of 5.75% to 6.25%, benzalkonium chloride in a concentration of 0.01%, and water. The pH of such a composition is preferably from 4.4. to 5.8, and the osmolality of such a composition is preferably from 625 to 875 mOsm/Kg, more preferably from 650 to 750 mOsm/Kg. If desired, such a composition may include an anti-redness agent such as, for example, naphazoline or naphazoline hydrochloride.

In another embodiment, the ophthalmic composition comprises ketotifen fumarate in a concentration of 0.0345%, glycerol in a concentration of 5.75% to 6.25%, benzalkonium chloride in a concentration of 0.01 %, and water. The pH of such a composition is preferably from 4.4. to 5.8, and the osmolality of such a composition is preferably from 625 to 875 mOsm/Kg, more preferably from 650 to 750 mOsm/Kg. If desired, such a composition may include an anti-redness agent such as, for example, naphazoline or naphazoline hydrochloride.

The ophthalmic compositions are useful for the treatment and temporary prevention of the signs and symptoms of allergic conjunctivitis, including itching of the eye and redness of the eye. Methods of treating allergic conjunctivitis comprise administering to a human subject suffering from or susceptible to allergic conjunctivitis an effective amount of an ophthalmic composition described herein.

Typically, the compositions are administered as drops, with one drop of the composition being applied to an eye of the subj ect suffering from or susceptible to allergic conjunctivitis two times per day, although more or less of the composition may be used in more or less frequent doses depending on multiple factors, including the makeup of the particular composition.

The ophthalmic compositions may also be useful for the treatment of dry eye disease, including inflammatory dry eye disease. Methods of treating dry eye disease comprise administering to a human subject suffering from dry eye disease an effective amount of an ophthalmic composition described herein.

The ophthalmic compositions may be formulated as single or multi dose units, with or without the use of a preservative, and may be manufactured by mixing the ingredients. The compositions may be packaged in single or multiple dosage forms, such as closed bottles, tubes, or other containers made from materials such as glass or plastic. In some embodiments, the packaging for the ophthalmic composition may be free or substantially free of antioxidant (e.g., as used in compositions described in U.S. Patent Nos. 6,455,547 and 6,576,649).

### EXAMPLES

The invention will be further explained by the following illustrative examples.

### Example 1

Three different formulations of the ophthalmic compositions described herein were prepared with glycerol concentrations of 4%, 5%, and 6%. A ketotifen fumarate product marketed by Novartis Ophthalmics, Inc. (East Hanover, NJ) under the name Zaditor^{™} was obtained for testing as a comparative product. Information concerning the ophthalmic compositions that were prepared and the comparative ketotifen fumarate product (as listed on the prescription information) is listed below in Table 1.

**Table I-Ophthalmic Compositions and Comparative Example**

| | **4% Glycerol** | **5% Glycerol** | **6% Glycerol** | **Comparative** |
|---|---|---|---|---|
| ketotifen | 0.0345% | 0.0345% | 0.0345% | 0.0345% (0.025%) |
| fumarate (ketotifen base) | (0.025%) | (0.025%) | (0.025%) | |
| Glycerol | 4.0% | 5.0% | 6.0% | not listed |
| benzalkonium chloride | 0.01% | 0.01% | 0.01% | 0.01% |
| pH | 5.68 | 5.73 | 5.74 | 4.4-5.8 |
| Osmolality (mOsm/Kg) | 454 | 561 | 689 | 210-300 |
| Other ingredients | purified water (balance), and sodium hydroxide and/or hydrochloric acid to adjust pH | purified water (balance), and sodium hydroxide and/or hydrochloric acid to adjust pH | purified water (balance), and sodium hydroxide and/or hydrochloric acid to adjust pH | purified water (balance), and sodium hydroxide and/or hydrochloric acid to adjust pH |

Two blinded tests were conducted in order to compare the comfort level of different solutions. In the first test, the comparative ketotifen fumarate product (i.e., Zaditor^{™}) was tested against the 4% glycerol composition. Each human subject randomly received a drop of one of the two compositions in the right eye and received a drop of the other composition in the left eye, but the subjects were not informed of the identity of the compositions. The subject then indicated which eye was more comfortable. The blinded comparative test resulted in more subjects indicating that the 4% glycerol composition was more comfortable than the comparative ketotifen fumarate product.

In the second test, the 4% glycerol composition was tested against the 6% glycerol composition. Each of six human subjects randomly received a drop of one of the two compositions in the right eye and received a drop of the other composition in the left eye, but the subjects were not informed of the identity of the compositions. After receiving each drop, the subject indicated the comfort of the eye receiving the drop on a scale of 1-10, with 10 being the most comfortable. The results of the second test are listed below in Table II, which shows that the 6% glycerol composition was more comfortable than the 4% glycerol composition.

**Table II-Comfort Indications of 4% Glycerol Composition vs. 6% Glycerol Composition**

| **Composition** | | | | | | | **Average** |
|---|---|---|---|---|---|---|---|
| **4% Glycerol** | 7 | 5 | 8 | 6 | 4 | 4 | 5.66 |
| **6% Glycerol** | 7 | 8 | 4 | 8 | 8 | 5 | 6.66 |

In conclusion, the results indicated that the 4% glycerol composition, which had an osmolality of 454 mOsm/Kg, was more comfortable in human eyes than the comparative ketotifen product, which has an osmolality listed on the corresponding prescription information of 210-300 mOsm/Kg. The results also indicated that the 6% glycerol composition, which had an osmolality of 689 mOsm/Kg, was more comfortable in human eyes than the 4% glycerol composition, which had an osmolality of 454 mOsm/Kg.

### Example 2

A formulation of an ophthalmic composition having 6% glycerol was compared to the ketotifen fumarate product marketed by Novartis Ophthalmics, Inc. (East Hanover, NJ) under the name Zaditor^{™} (described as the comparative product in Table I above). The formulation (NFKF) of the ophthalmic composition comprised 6% glycerol, 0.0345% ketotifen fumarate (0.025% ketotifen), benzalkonium chloride, NaOH and/or HCl to adjust pH, and water. The NFKF formulation and the Zaditor product were tested in a conjunctival allergen challenge (CAC) model as described below.

### Protocol

The study involved four visits over a five week period.

During Visit 1 (Day -21 ± 3), a CAC was performed on each subject in order to determine (through titration) an appropriate dose of allergen to induce a moderate ocular allergic reaction. Increasingly concentrated doses of allergen were instilled bilaterally at ten-minute intervals until a positive ocular allergic reaction was elicited.

During Visit 2 (Day -14± 3), one drop of the allergen solution, of the same type and dose that elicited a positive reaction at Visit 1, was administered bilaterally to the subjects to confirm and reproduce the ocular allergic reaction. Assessment of itching was made by the subject at 3, 5, and 7 minutes following allergen challenge. Assessments of redness (hyperemia) were graded by the investigator at 7, 15 and 20 minutes post-challenge. Subjects failing to react positively in both eyes in at least two out of the three timepoints within the 20 minute interval were discontinued from the study.

At Visit 3 (Day 0± 3), subjects who met the inclusion/exclusion criteria were randomized by eye to one of the following treatment groups:
NFKF/NFKF (n=30)
Zaditor/ Zaditor (n=30)
NFKF/Vehicle (placebo) (n=20)
Zaditor/ Vehicle (placebo) (n=20).
Subjects received pre-treatment with study medication (NFKF, Zaditor, placebo) in each eye according to the randomization scheme. Eight hours after drug instillation each subject received one drop of the allergen solution bilaterally, of the same allergen and dose that elicited a positive reaction at Visits 1 and 2. Assessment of itching was made by the subject at 3, 5, and 7 minutes following allergen challenge. Assessments of hyperemia (conjunctival, ciliary, and episcleral) were graded by the investigator at 7, 15 and 20 minutes post-challenge.

At Visit 4 (Day 14± 3), subjects received pre-treatment with study medication (NFKF, Zaditor, placebo) in each eye according to the randomization scheme. Fifteen (15) minutes after drug instillation each subject was administered one drop of the allergen solution bilaterally, of the same allergen and dose that elicited a positive reaction at Visits 1 and 2. Assessment of itching was made by the subject at 3, 5, and 7 minutes following allergen challenge. Assessments of hyperemia (conjunctival, ciliary, and episcleral) were graded by the investigator at 7, 15 and 20 minutes post-challenge.

### Results

Statistical summaries of the ocular itching scores at Visits 3 and 4 by treatment group (NFKF, Zaditor, or placebo) are presented below in Tables III and IV, respectively. Statistical summaries of the conjunctival redness scores at Visits 3 and 4 by treatment group are presented below in Tables V and VI, respectively. Statistical summaries of the ciliary redness scores at Visits 3 and 4 by treatment group are presented below in Tables VII and VIII, respectively. Statistical summaries of the episcleral redness scores at Visits 3 and 4 by treatment group are presented below in Tables IX and X, respectively.

Itching was evaluated on a 0 to 4 scale, allowing for half increment scores, where 0 indicates no itching and 4 indicates severe itching. Conjunctival, ciliary, and episcleral redness were all evaluated on a 0 to 4 scale, allowing for half increment scores, where 0 indicates no redness and 4 indicates severe redness.

Each of Tables III-X includes various comparisons ofNFKF and Zaditor to placebo at different timepoints. Each of Tables III and IV also include comparisons of NFKF to Zaditor at different time points. The comparisons include differences between the mean values for the groups compared as well as p-values (calculated using Fisher's Exact test).

As shown in Tables III and IV, the comparisons of NFKF to Zaditor show bioequivalence of NFKF to Zaditor with respect to reduction in ocular itching. The comparisons of NFKF and Zaditor to placebo in Tables III and IV show that the reduction in ocular itching due to both formulations was statistically significant at every measured time point.

As illustrated in each of Tables V-X, the NFKF formulation having 6% glycerol and 0.025% ketotifen unexpectedly showed better redness scores than the Zaditor ketotifen product. The comparisons of NFKF and Zaditor to placebo with respect to reduction in redness in Tables V-X showed more examples of statistical significance with respect to NFKF than Zaditor.

**Table III: Summary of Ocular Itching Scores by Eye Treatment at Visit 3**

| Time Point | | NFKF | Zaditor | Placebo |
|---|---|---|---|---|
| | | | | |
| Visit 3 (Day 0) | | | | |
| Pre-CAC | n | 90 | 81 | 45 |
| | Mean | 0.00 | 0.00 | 0.00 |
| | Median | 0.0 | 0.0 | 0.0 |
| | SD | 0.000 | 0.000 | 0.000 |
| | (Min,Max) | (0.0,0.0) | (0.0,0.0) | (0.0,0.0) |
| | | | | |
| 3 Min Post-CAC | *n* | 90 | 81 | 45 |
| | Mean | 1.34 | 1.41 | 2.44 |
| | Median | 1.5 | 1.5 | 2.5 |
| | SD | 1.040 | 1.013 | 0.867 |
| | (Min,Max) | (0.0,4.0) | (0.0,3.5) | (0.0,3.5) |
| | | | | |
| Comparison with Placebo | Diff | -1.240 | -1.158 | |
| | | | | |
| | p-value | <0.0001 | <0.0001 | |
| | | | | |
| Comparison of NFKF with Zaditor | Diff | -0.082 | | |
| | | | | |
| | p-value | 0.623 | | |
| | | | | |
| 5 Min Post-CAC | n | 90 | 81 | 45 |
| | Mean | 1.37 | 1.43 | 2.59 |
| | Median | 1.0 | 1.5 | 2.5 |
| | SD | 1.040 | 1.018 | 0.861 |
| | (Min,Max) | (0.0,3.0) | (0.0,3.5) | (0.5,4.0) |
| | | | | |
| Comparison with Placebo | Diff | -1.321 | -1.265 | |
| | | | | |
| | p-value | <0.0001 | <0.0001 | |
| | | | | |
| Comparison of NFKF with Zaditor | Diff | -0.055 | | |
| | | | | |
| | p-value | 0.737 | | |
| | | | | |
| 7 Min Post-CAC | n | 90 | 81 | 45 |
| | Mean | 1.23 | 1.35 | 2.52 |
| | Median | 1.0 | 1.5 | 2.5 |
| | SD | 1.036 | 1.044 | 0.866 |
| | (Min,Max) | (0.0,3.0) | (0-0,3.5) | (0.5,4.0) |
| | | | | |
| Comparison with Placebo | Diff | -1.314 | -1.305 | |
| | | | | |
| | p-value | <0.0001 | <0.0001 | |
| | | | | |
| Comparison of NFKF with Zaditor | Diff | -0.009 | | |
| | | | | |
| | p-value | 0.956 | | |

**Table IV: Summary of Ocular Itching Scores by Eye Treatment at Visit 4**

| Time Point | | NFKF | Zaditor | Placebo |
|---|---|---|---|---|
| | | | | |
| Visit 4 (Day 14) | | | | |
| Pre-CAC | n | 90 | 74 | 45 |
| | Mean | 0.00 | 0.00 | 0.00 |
| | Median | 0.0 | 0.0 | 0.0 |
| | SD | 0.000 | 0.000 | 0.000 |
| | (Min,Max) | (0.0,0.0) | (0.0,0.0) | (0.0,0.0) |
| | | | | |
| 3 Min Post-CAC | n | 89 | 73 | 44 |
| | Mean | 0.46 | 0.71 | 2.23 |
| | Median | 0.0 | 0.5 | 2.5 |
| | SD | 0.632 | 0.837 | 0.866 |
| | (Min,Max) | (0.0,2.5) | (0.0,3.0) | (0.0,4.0) |
| | | | | |
| Comparison with Placebo | Diff | -1.649 | -1.481 | |
| | | | | |
| | p-value | <0.0001 | <0.0001 | |
| | | | | |
| Comparison of NFKF with Zaditor | Diff | -0.168 | | |
| | | | | |
| | p-value | 0.202 | | |
| | | | | |
| 5 Min Post-CAC | n | 89 | 73 | 44 |
| | Mean | 0.61 | 0.72 | 2.33 |
| | Median | 0.5 | 0.5 | 2.5 |
| | SD | 0.757 | 0.782 | 0.876 |
| | (Min,Max) | (0.0,2.5) | (0.0,3.0) | (0.0,4.0) |
| | | | | |
| Comparison with Placebo | Diff | -1.663 | -1.622 | |
| | | | | |
| | p-value | <0.0001 | <0.0001 | |
| | | | | |
| Comparison of NFKF with Zaditor | Diff | -0.041 | | |
| | | | | |
| | p-value | 0.764 | | |
| | | | | |
| 7 Min Post-CAC | n | 89 | 73 | 44 |
| | Mean | 0.53 | 0.66 | 2.18 |
| | Median | 0.5 | 0.5 | 2.0 |
| | SD | 0.667 | 0.795 | 0.947 |
| | (Min,Max) | (0.0,2.5) | (0.0,3.0) | (0.0,3.5) |
| | | | | |
| Comparison with Placebo | Diff | -1.594 | -1.565 | |
| | | | | |
| | p-value | <0.0001 | <0.0001 | |
| | | | | |
| Comparison of NFKF with Zaditor | Diff | -0.029 | | |
| | | | | |
| | p-value | 0.828 | | |

**Table V: Summary of Conjuctival Redness Scores by Eye Treatment at Visit 3**

| Time Point | | NFKF | Zaditor | Placebo |
|---|---|---|---|---|
| | | | | |
| Visit 3 (Day 0) | | | | |
| Pre-CAC | n | 90 | 81 | 45 |
| | Mean | 0.29 | 0.24 | 0.29 |
| | Median | 0.3 | 0.0 | 0.0 |
| | SD | 0.326 | 0.307 | 0.328 |
| | (Min,Max) | (0.0,1.0) | (0.0,1.0) | (0.0,1.0) |
| | | | | |
| 7 Min Post-CAC | n | 90 | 81 | 45 |
| | Mean | 1.40 | 1.54 | 1.70 |
| | Median | 1.5 | 2.0 | 2.0 |
| | SD | 0.724 | 0.676 | 0.558 |
| | (Min,Max) | (0.0,2.5) | (0.0,3.0) | (0.5,3.0) |
| | | | | |
| Comparison with Placebo | Diff | -0.277 | -0.151 | |
| | | | | |
| | p-value | 0.006 | 0.141 | |
| | | | | |
| 15 Min Post-CAC | n | 90 | 81 | 45 |
| | Mean | 1.68 | 1.81 | 1.88 |
| | Median | 2.0 | 2.0 | 2.0 |
| | SD | 0.697 | 0.664 | 0.641 |
| | (Min,Max) | (0.0,3.0) | (0.0,3.0) | (0.5,3.0) |
| | | | | |
| Comparison with Placebo | Diff | -0.172 | -0.089 | |
| | | | | |
| | p-value | 0.094 | 0.399 | |
| | | | | |
| 20 Min Post-CAC | n | 90 | 81 | 45 |
| | Mean | 1.64 | 1.76 | 1.84 |
| | Median | 2.0 | 2.0 | 2.0 |
| | SD | 0.708 | 0.676 | 0.681 |
| | (Min,Max) | (0.0,2.5) | (0.0,3.0) | (0.0,3.0) |
| | | | | |
| Comparison with Placebo | Diff | -0.185 | -0.146 | |
| | | | | |
| | p-value | 0.072 | 0.171 | |

**Table VI: Summary of Conjuctival Redness Scores by Eye Treatment at Visit 4**

| Time Point | | NFKF | Zaditor | Placebo |
|---|---|---|---|---|
| | | | | |
| Visit 4 (Day 14) | | | | |
| Pre-CAC | n | 90 | 75 | 45 |
| | Mean | 0.21 | 0.23 | 0.28 |
| | Median | 0.0 | 0.0 | 0.0 |
| | SD | 0.366 | 0.388 | 0.407 |
| | (Min,Max) | (0.0,2.0) | (0.0,2.0) | (0.0,2.0) |
| | | | | |
| 7 Min Post-CAC | n | 89 | 73 | 44 |
| | Mean | 1.10 | 1.14 | 1.72 |
| | Median | 1.0 | 1.0 | 2.0 |
| | SD | 0.822 | 0.867 | 0.742 |
| | (Min,Max) | (0.0,2.5) | (0.0,2.5) | (0.0,3.0) |
| | | | | |
| Comparison with Placebo | Diff | -0.508 | -0.462 | |
| | | | | |
| | p-value | <0.0001 | <0.0001 | |
| | | | | |
| 15 Min Post-CAC | n | 89 | 73 | 44 |
| | Mean | 1.32 | 1.47 | 1.85 |
| | Median | 1.5 | 2.0 | 2.0 |
| | SD | 0.802 | 0.866 | 0.744 |
| | (Min,Max) | (0.0,2.5) | (0.0,3.0) | (0.0,3.0) |
| | | | | |
| Comparison with Placebo | Diff | -0.436 | -0.303 | |
| | | | | |
| | p-value | <0.0001 | 0.017 | |
| | | | | |
| 20 Min Post-CAC | n | 89 | 73 | 44 |
| | Mean | 1.32 | 1.53 | 1.82 |
| | Median | 1.5 | 2.0 | 2.0 |
| | SD | 0.820. | 0.889 | 0.786 |
| | (Min,Max) | (0.0,3.0) | (0.0,3.0) | (0.0,3.0) |
| | | | | |
| Comparison with Placebo | Diff | -0.378 | -0.282 | |
| | | | | |
| | p-value | 0.001 | 0.021 | |

**Table VII: Summary of Ciliary Redness Scores by Eye Treatment at Visit 3**

| Time Point | | NFKF | Zaditor | Placebo |
|---|---|---|---|---|
| | | | | |
| Visit 3 (Day 0) | | | | |
| Pre-CAC | n | 90 | 80 | 45 |
| | Mean | 0.34 | 0.29 | 0.38 |
| | Median | 0.5 | 0.0 | 0.5 |
| | SD | 0.366 | 0.363 | 0.401 |
| | (Min,Max) | (0.0,1.0) | (0.0,1.0) | (0.0,1.0) |
| | | | | |
| 7 Min Post-CAC | n | 90 | 81 | 45 |
| | Mean | 1.42 | 1.61 | 1.73 |
| | Median | 1.5 | 1.5 | 2.0 |
| | SD | 0.782 | 0.685 | 0.627 |
| | (Min,Max) | (0.0,3.0) | (0.5,3.0) | (0.5,3.0) |
| | | | | |
| Comparison with Placebo | Diff | -0.315 | -0.206 | |
| | | | | |
| | p-value | 0.001 | 0.038 | |
| | | | | |
| 15 Min Post-CAC | n | 90 | 81 | 45 |
| | Mean | 1.67 | 1.91 | 1.97 |
| | median | 2.0 | 2.0 | 2.0 |
| | SD | 0.761 | 0.715 | 0.694 |
| | (Min,Max) | (0.0,3.0) | (0.5,3.5) | (0.5,3.5) |
| | | | | |
| Comparison with Placebo | Diff | -0.274 | -0.127 | |
| | | | | |
| | p-value | 0.002 | 0.173 | |
| | | | | |
| 20 Min Post-CAC | n | 90 | 81 | 45 |
| | Mean | 1.64 | 1.88 | 1.91 |
| | Median | 2.0 | 2.0 | 2.0 |
| | SD | 0.769 | 0.690 | 0.693 |
| | (Min,Max) | (0.0,3.0) | (0.5,3.5) | (0.5,3.0) |
| | | | | |
| Comparison with Placebo | Diff | -0.224 | -0.077 | |
| | | | | |
| | p-value | 0.016 | 0.421 | |

**Table VIII: Summary of Ciliary Redness Scores by Eye Treatment at Visit 4**

| Time Point | | NFKF | Zaditor | Placebo |
|---|---|---|---|---|
| Visit 4 (Day 14) | | | | |
| Pre-CAC | n | 89 | 75 | 45 |
| | Mean | 0.12 | 0.14 | 0.16 |
| | Median | 0.0 | 0.0 | 0.0 |
| | SD | 0.239 | 0.335 | 0.278 |
| | (Min,Max) | (0.0,1.0) | (0.0,2.0) | (0.0,1.0) |
| | | | | |
| 7 Min Post-CAC | n | 89 | 73 | 44 |
| | Mean | 0.89 | 1.04 | 1.63 |
| | Median | 0.5 | 1.0 | 2.0 |
| | SD | 0.768 | 0.824 | 0.724 |
| | (Min,Max) | (0.0,2.5) | (0.0,2.5) | (0.0,3.0) |
| | | | | |
| Comparison with Placebo | Diff | -0.662 | -0.494 | |
| | | | | |
| | p-value | <0.0001 | 0 | |
| | | | | |
| 15 Min Post-CAC | n | 89 | 73 | 44 |
| | Mean | 1.13 | 1.33 | 1.75 |
| | Median | 1.0 | 1.5 | 2.0 |
| | SD | 0.815 | 0.792 | 0.735 |
| | (Min,Max) | (0.0,2.5) | (0.0,3.0) | (0.0,3.0) |
| | | | | |
| Comparison with Placebo | Diff | -0.540 | -0.329 | |
| | | | | |
| | p-value | <0.0001 | 0.004 | |
| | | | | |
| 20 Min Post-CAC | n | 89 | 73 | 44 |
| | Mean | 1.16 | 1.40 | 1.77 |
| | Median | 1.0 | 1.5 | 2.0 |
| | SD | 0.816 | 0.833 | 0.774 |
| | (Min,Max) | (0.0,2.5) | (0.0,3.0) | (0.0,3.0) |
| | | | | |
| Comparison with Placebo | Diff | -0.522 | -0.371 | |
| | | | | |
| | p-value | <0.0001 | 0.002 | |

**Table IX: Summary of Episcleral Redness Scores by Eye Treatment at Visit 3**

| Time Point | | NFKF | Zaditor | Placebo |
|---|---|---|---|---|
| | | | | |
| Visit 3 (Day 0) | | | | |
| Pre-CAC | n | 90 | 81 | 45 |
| | Mean | 0.28 | 0.29 | 0.30 |
| | Median | 0.0 | 0.0 | 0.0 |
| | SD | 0.327 | 0.343 | 0.375 |
| | (Min,Max) | (0.0,1.0) | (0.0,1.0) | (0.0,1.0) |
| | | | | |
| 7 Min Post-CAC | n | 90 | 80 | 45 |
| | Mean | 1.50 | 1.68 | 1.78 |
| | Median | 1.5 | 2.0 | 2.0 |
| | SD | 0.757 | 0.713 | 0.580 |
| | (Min,Max) | (0.0,3.0) | (0.0,3.0) | (0.5,3.0) |
| | | | | |
| Comparison with Placebo | Diff | -0.230 | -0.048 | |
| | | | | |
| | p-value | 0.026 | 0.652 | |
| | | | | |
| 15 Min Post-CAC | n | 90 | 81 | 45 |
| | Mean | 1.75 | 1.94 | 1.99 |
| | Median | 2.0 | 2.0 | 2.0 |
| | SD | 0.720 | 0.686 | 0.678 |
| | (Min,Max) | (0.0,3.0) | (0.5,3.5) | (0.0,3.0) |
| | | | | |
| Comparison with Placebo | Diff | -0.153 | -0.006 | |
| | | | | |
| | p-value | 0.124 | 0.95 | |
| | | | | |
| 20 Min Post-CAC | n | 90 | 81 | 45 |
| | Mean | 1.73 | 1.89 | 1.91 |
| | Median | 2.0 | 2.0 | 2.0 |
| | SD | 0.754 | 0.703 | 0.709 |
| | (Min,Max) | (0.0,3.0) | (0.0,3.5) | (0.0,3.0) |
| | | | | |
| Comparison with Placebo | Diff | -0.124 | -0.044 | |
| | | | | |
| | p-value | 0.221 | 0.678 | |

**Table X: Summary of Episcleral Redness Scores by Eye Treatment at Visit 4**

| Time Point | | NFKF | Zaditor | Placebo |
|---|---|---|---|---|
| | | | | |
| Visit4 (Day 14) | | | | |
| Pre-CAC | n | 90 | 75 | 45 |
| | Mean | 0.14 | 0.21 | 0.20 |
| | Median | 0.0 | 0.0 | 0.0 |
| | SD | 0.335 | 0.395 | 0.405 |
| | (Min,Max) | (0.0, 2.0) | (0.0,2.0) | (0.0, 2.0) |
| | | | | |
| 7 Min Post-CAC | n | 89 | 73 | 44 |
| | Mean | 1.07 | 1.23 | 1.75 |
| | Median | 1.0 | 1.0 | 2.0 |
| | SD | 0.850 | 0.862 | 0.803 |
| | (Min,Max) | (0.0,3.0) | (0.0,2.5) | (0.0,3.0) |
| | | | | |
| Comparison with Placebo | Diff | -0.580 | -0.454 | |
| | | | | |
| | p-value | <0.0001 | 0.001 | |
| | | | | |
| 15 Min Post-CAC | n | 89 | 73 | 44 |
| | Mean | 1.40 | 1.55 | 1.89 |
| | Median | 1.5 | 2.0 | 2.0 |
| | SD | 0.853 | 0.896 | 0.761 |
| | (Min,Max) | (0.0,2.5) | (0.0,3.0) | (0.0,3.0) |
| | | | | |
| Comparison with Placebo | Diff | -0.378 | -0.249 | |
| | | | | |
| | p-value | 0.003 | 0.054 | |
| | | | | |
| 20 Min Post-CAC | n | 89 | 73 | 44 |
| | Mean | 1.48 | 1.55 | 2.00 |
| | Median | 2.0 | 2.0 | 2.0 |
| | SD | 0.898 | 0.923 | 0.842 |
| | (Min,Max) | (0.0,3.0) | (0.0,3.0) | (0.0,3.0) |
| | | | | |
| Comparison with Placebo | Diff | -0.392 | -0.382 | |
| | | | | |
| | p-value | 0.002 | 0.003 | |

## Claims

1. An ophthalmic composition comprising:
(a) ketotifen or a ketotifen salt in a concentration of from 0.01 % w/v to 0.05% w/v;
(b) a non-ionic tonicity agent in a concentration such that the composition has an osmolality of from 400 to 875 mOsm/Kg; and
(c) water.

2. The composition of claim 1, wherein the non-ionic tonicity agent is glycerol.

3. The composition of claim 2, wherein the ophthalmic composition comprises glycerol in a concentration from 3.5% w/v to 7% w/v.

4. The composition of claim 3, wherein the concentration of the glycerol is from 4.5% w/v to 7% w/v.

5. The composition of claim 3, wherein the concentration of the glycerol is from 5.75% w/v to 6.25% w/v.

6. The composition of claim 3, wherein the composition consists essentially of ketotifen fumarate in a concentration of from 0.03% w/v to 0.04% w/v, glycerol in a concentration of from 5.75% w/v to 6.25% w/v, and water.

7. The composition of any one of the preceding claims, wherein the composition has an osmolality of from 425 to 775 mOsm/Kg.

8. The composition of any one of the preceding claims, wherein the composition has an osmolality of from 550 to 750 mOsm/Kg.

9. The composition of any one of the preceding claims, wherein the composition has an osmolality of from 600 to 725 mOsm/Kg.

10. The composition of any one of the preceding claims, wherein the composition has an osmolality of from 650 to 700 mOsm/Kg.

11. The composition of claim 2, wherein the ophthalmic composition comprises glycerol in a concentration greater than 3.5% w/v such that the composition has an osmolality of from 400 to 875 mOsm/Kg.

12. The composition of claim 11, wherein the concentration of the glycerol is greater than 4.5% w/v.

13. The composition of claim 11, wherein the concentration of the glycerol is greater than 5.5% w/v..

14. The composition of claim 3 or 11, wherein the concentration of the glycerol is from 5% w/v to 7% w/v.

15. The composition of claim 3 or 11, wherein the concentration of the glycerol is from 5.5% w/v to 6.5% w/v.

16. The composition of claim 11, wherein the composition consists essentially of ketotifen fumarate in a concentration of from 0.03% w/v to 0.04% w/v, glycerol in a concentration of from 5.5% w/v to 6.5% w/v such that the composition has an osmolality of from 650 to 700 mOsm/Kg, and water.

17. The composition of any one of the preceding claims, further comprising an anti-redness agent.

18. The composition of claim 17, wherein:
the concentration of the ketotifen fumarate is 0.0345% w/v;
the concentration of the glycerol is from 5.75% w/v to 6.25% w/v;
the composition includes benzalkonium chloride in a concentration of 0.01 % w/v; and
the pH of the composition is from 4.4 to 5.8.

19. The composition of any one of the preceding claims, wherein the ketotifen or ketotifen salt is ketotifen fumarate.

20. The composition of claim 19, wherein the concentration of the ketotifen fumarate is from 0.03% w/v to 0.04% w/v.

21. The composition of any one of the preceding claims, wherein the concentration of the ketotifen or ketotifen salt is such that the concentration of ketotifen base in the composition is from 0.02% w/v to 0.03% w/v.

22. An ophthalmic composition according to any one of the preceding claims for treating allergic conjunctivitis.

23. An ophthalmic composition according to any one of claim 1 to 21 for treating dry eye disease.

## Patentansprüche

1. Eine ophthalmische Zusammensetzung beinhaltend:
(a) Ketotifen oder ein Ketotifensalz in einer Konzentration von 0,01% m/V bis 0,05% m/V;
(b) ein nichtionisches Tonizitätsmittel in einer Konzentration, so dass die Zusammensetzung eine Osmolalität von 400 bis 875 mOsm/kg aufweist, und
(c) Wasser.

2. Die Zusammensetzung gemäß Anspruch 1, wobei das nichtionische Tonizitätsmittel Glycerin ist.

3. Die Zusammensetzung gemäß Anspruch 2, wobei die ophthalmische Zusammensetzung Glycerin in einer Konzentration von 3,5% m/V bis 7% m/V enthält.

4. Die Zusammensetzung gemäß Anspruch 3, wobei die Glycerinkonzentration bei 4,5% m/V bis 7% m/V liegt.

5. Die Zusammensetzung gemäß Anspruch 3, wobei die Glycerinkonzentration bei 5,75% m/V bis 6,25% m/V liegt.

6. Die Zusammensetzung gemäß Anspruch 3, wobei die Zusammensetzung im Wesentlichen aus Ketotifenfumarat in einer Konzentration von 0,03% m/V bis 0,04% m/V, Glycerin in einer Konzentration von 5,75% m/V bis 6,25% m/V und Wasser besteht.

7. Die Zusammensetzung gemäß einem der vorhergehenden Ansprüche, wobei die Zusammensetzung eine Osmolalität von 425 bis 775 mOsm/kg aufweist.

8. Die Zusammensetzung gemäß einem der vorhergehenden Ansprüche, wobei die Zusammensetzung eine Osmolalität von 550 bis 750 mOsm/kg aufweist.

9. Die Zusammensetzung gemäß einem der vorhergehenden Ansprüche, wobei die Zusammensetzung eine Osmolalität von 600 bis 725 mOsm/kg aufweist.

10. Die Zusammensetzung gemäß einem der vorhergehenden Ansprüche, wobei die Zusammensetzung eine Osmolalität von 650 bis 700 mOsm/kg aufweist.

11. Die Zusammensetzung gemäß Anspruch 2, wobei die ophthalmische Zusammensetzung eine höhere Glycerinkonzentration als 3,5% m/V beinhaltet, so dass die Zusammensetzung eine Osmolalität von 400 bis 875 mOsm/kg aufweist.

12. Die Zusammensetzung gemäß Anspruch 11, wobei die Glycerinkonzentration größer als 4,5% m/V ist.

13. Die Zusammensetzung gemäß Anspruch 11, wobei die Glycerinkonzentration größer als 5,5% m/V ist.

14. Die Zusammensetzung gemäß Anspruch 3 oder 11, wobei die Glycerinkonzentration bei 5% m/V bis 7% m/V liegt.

15. Die Zusammensetzung gemäß Anspruch 3 oder 11, wobei die Glycerinkonzentration bei 5,5% m/V bis 6,5% m/V liegt.

16. Die Zusammensetzung gemäß Anspruch 11, wobei die Zusammensetzung im Wesentlichen aus Ketotifenfumarat in einer Konzentration von 0,03% m/V bis 0,04% m/V, Glycerin in einer Konzentration von 5,5% m/V bis 6,5% m/V und Wasser besteht, so dass die Zusammensetzung eine Osmolalität von 650 bis 700 mOsm/kg aufweist.

17. Die Zusammensetzung gemäß einem der vorhergehenden Ansprüche, die ferner ein Mittel gegen Rötungen enthält.

18. Die Zusammensetzung gemäß Anspruch 17, wobei:
die Konzentration des Ketotifenfumarats 0,0345% m/V ist,
die Glycerinkonzentration bei 5,75% m/V bis 6,25% m/V liegt,
die Zusammensetzung Benzalkoniumchlorid in einer Konzentration von 0,01% m/V enthält, und
der pH-Wert der Zusammensetzung bei 4,4 bis 5,8 liegt.

19. Die Zusammensetzung gemäß einem der vorhergehenden Ansprüche, wobei das Ketotifen oder Ketotifensalz Ketotifenfumarat ist.

20. Die Zusammensetzung gemäß Anspruch 19, wobei die Ketotifenfumaratkonzentration bei 0,03% m/V bis 0,04% m/V liegt.

21. Die Zusammensetzung gemäß einem der vorhergehenden Ansprüche, wobei die Konzentration von Ketotifen oder des Ketotifensalzes derart ist, dass die Konzentration der Ketotifenbase in der Zusammensetzung bei 0,02% m/V bis 0,03% m/V liegt.

22. Eine ophthalmische Zusammensetzung gemäß einem der vorhergehenden Ansprüche zur Behandlung von allergischer Konjunktivitis.

23. Eine ophthalmische Zusammensetzung gemäß einem der Ansprüche 1 bis 21 1 zur Behandlung von trockenen Augen.

## Revendications

1. Composition ophtalmique, qui comprend :
(a) du kétotifène ou un sel de kétotifène en une concentration de 0,01 % p/v à 0,05 % p/v ;
(b) un agent de tonicité non ionique en une concentration telle que la composition a une osmolalité comprise entre 400 et 875 mOsm/kg ; et
(c) de l'eau.

2. Composition selon la revendication 1, dans laquelle l'agent de tonicité non ionique est le glycérol.

3. Composition selon la revendication 2, dans laquelle la composition ophtalmique comprend du glycérol en une concentration comprise entre 3,5 % p/v et 7 % p/v.

4. Composition selon la revendication 3, dans laquelle la concentration du glycérol est comprise entre 4,5 % p/v et 7 % p/v.

5. Composition selon la revendication 3, dans laquelle la concentration du glycérol est comprise entre 5,75 % p/v et 6,25 % p/v.

6. Composition selon la revendication 3, dans laquelle la composition est essentiellement constituée par du kétotifène fumarate en une concentration comprise entre 0,03 % p/v et 0,04 % p/v ; du glycérol en une concentration comprise entre 5,75 % p/v et 6,25 % p/v, et de l'eau.

7. Composition selon l'une quelconque des revendications précédentes, dans laquelle la composition a une osmolalité de 425 à 775 mOsm/kg.

8. Composition selon l'une quelconque des revendications précédentes, dans laquelle la composition a une osmolalité de 550 à 750 mOsm/kg.

9. Composition selon l'une quelconque des revendications précédentes, dans laquelle la composition a une osmolalité de 600 à 725 mOsm/kg.

10. Composition selon l'une quelconque des revendications précédentes, dans laquelle la composition a une osmolalité de 650 à 700 mOsm/kg.

11. Composition selon la revendication 2, dans laquelle la composition ophtalmique comprend du glycérol en une concentration supérieure à 3,5 % p/v de telle manière que la composition a une osmolalité comprise entre 400 et 875 mOsm/kg.

12. Composition selon la revendication 11, dans laquelle la concentration du glycérol est supérieure à 4,5 % p/v.

13. Composition selon la revendication 11, dans laquelle la concentration du glycérol est supérieure à 5,5 % p/v.

14. Composition selon la revendication 3 ou 11, dans laquelle la concentration du glycérol est comprise entre 5 % p/v et 7 % p/v.

15. Composition selon la revendication 3 ou 11, dans laquelle la concentration du glycérol est comprise entre 5,5 % p/v et 6,5 % p/v.

16. Composition selon la revendication 11, dans laquelle la composition est essentiellement constituée par du kétotifène fumarate en une concentration de 0,03 % p/v à 0,04 % p/v ; du glycérol en une concentration de 5,5 % p/v à 6,5 % p/v de telle manière que la composition a une osmolalité de 650 à 700 mOsm/kg, et de l'eau.

17. Composition selon l'une quelconque des revendications précédentes, comprenant en outre un agent anti-rougeur.

18. Composition selon la revendication 17, dans laquelle :
la concentration du kétotifène fumarate est de 0,0345 % p/v ;
la concentration du glycérol est de 5,75 % p/v à 6,25 % p/v ;
la composition comprend du chlorure de benzalkonium en une concentration de 0,01 % p/v ; et
le pH de la composition est compris entre 4,4 et 5,8

19. Composition selon l'une quelconque des revendications précédentes, dans laquelle le kétotifène ou le sel de kétotifène est le fumarate de kétotifène.

20. Composition selon la revendication 19, dans laquelle la concentration du fumarate de kétotifène est comprise entre 0,03 % p/v et 0,04 % p/v.

21. Composition selon l'une quelconque des revendications précédentes, dans laquelle la concentration du kétotifène ou du sel de kétotifène est telle que la concentration de la base de kétotifène dans la composition est comprise entre 0,02 % p/v et 0,03 % p/v.

22. Composition ophtalmique selon l'une quelconque des revendications précédentes destinée au traitement de la conjonctivite allergique.

23. Composition ophtalmique selon l'une quelconque des revendications 1 à 21, destinée au traitement de sécheresse oculaire.
